(19) Europäisches Patentamt
European Patent Office
Office européen des brevets

(11) **EP 4 717 302 A1**

(12) **EUROPEAN PATENT APPLICATION**

(43) Date of publication:
**01.04.2026 Bulletin 2026/14**

(21) Application number: **24202738.1**

(22) Date of filing: **26.09.2024**

(51) International Patent Classification (IPC):
**A61N 1/375** (2006.01)      **H01B 7/04** (2006.01)
**A61N 1/36** (2006.01)       **A61N 1/05** (2006.01)
**H01B 7/18** (2006.01)

(52) Cooperative Patent Classification (CPC):
**A61N 1/375; H01B 7/048;** A61N 1/05; A61N 1/3605;
H01B 7/18

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BIOTRONIK SE & Co. KG**
**12359 Berlin (DE)**

(72) Inventors:
• **RUMP, Jens**
**12049 Berlin (DE)**
• **BUCHNER, Dagmar**
**12435 Berlin (DE)**

(74) Representative: **Biotronik Corporate Services SE**
**Corporate Intellectual Property**
**Sieversufer 9**
**12359 Berlin (DE)**

(54) **IMPLANTABLE MEDICAL LEAD**

(57)    The invention refers to an implantable medical lead that may be cost-efficiently designed for different use cases and provides excellent mechanical properties. The implantable medical lead comprises an electrically insulating tubular lead body having an internal lumen accommodating at least two conductors, wherein each conductor is configured to electrically connect one electrode located at the distal end of the lead and an electrical connector assembly at the proximal end of the lead, wherein each conductor comprises an electrically conductive core and an electrically insulating sleeve, wherein in a cross section of the electrically insulating sleeve of at least one conductor of the at least two conductors forms an inner circumference and an outer circumference, wherein the inner circumference and the outer circumference have a substantially elliptical shape exhibiting an eccentricity $\varepsilon$ in the range $0{,}4 \leq \varepsilon 1 \leq 0{,}85$.

FIG. 2

EP 4 717 302 A1

**Description**

The invention generally relates to an implantable medical lead for electrical therapy like defibrillation, pacing, spinal cord stimulation, deep brain stimulation or neurostimulation or for electrical sensing of bodily parameters.

Medical leads implanted in a patient's body for electrical cardioversion or pacing of the heart are generally known in the art. In particular, electrically conducting leads may be implanted in or about the heart to reverse (i.e., defibrillate or cardiovert) certain life-threatening arrhythmias or to stimulate contraction (pacing) of the heart. Electrical energy is transmitted from a pulse generator which can be electrically connected to the lead. Such transmitted electrical energy is applied to the heart via the lead to return the heart to normal rhythm or to stimulate the heart. Leads have also been used to sense conditions, materials or events (generally referred to as "sense" or "sensing") in the body, such as electrical potential in the atrium or ventricle of the heart. For that such medical lead may be connected to a sensing and/or data processing device using a connector assembly at the proximal end of the medical lead. Alternatively, the sensed signals may be transmitted to and processed by the pulse generator, and, for example, used for pacing control. Further, implantable medical leads may be used in connection with spinal cord stimulation (SCS), deep brain stimulation (DBS) or neurostimulation devices.

Implantable medical leads represent the electrical link between the stimulation signal generator or processing unit for measured signals and the patient's body tissue which is to be treated or sensed. Accordingly, the medical lead must be mechanically and electrically reliably connected to the patient's body tissue at a pre-defined target location.

During use and implantation an implantable medical lead is subject to various mechanical forces. A medical lead has to endure rotational, bending, tensile, and compressive forces, minimize friction with the inner surfaces of the catheter but also needs to allow a degree of flexibility to protect the electrical conducting material against damage.

The above use cases for implantable medical leads often require that the lead comprises multiple electrically conducting filaments. The single electrically conducting filament of such medical lead that may comprise a single strand or multiple strands is electrically insulated relative to another electrically conducting filament by electrically insulating material. This material having a suitable wall thickness does not only provide required electrical properties (i.e. electrical insulation) but also substantially affects the mechanical properties of the implantable medical lead. In many cases, high wall thickness of electrically insulating material and compressible lumen within the lead are an advantage. However, if high voltages are required, a straight or helical filament comprising a silver core is typically utilized. For such filament type, a sleeve is commonly crimped at the lead thereby providing an electrical connection to the filament to create an electrode portion. As a result, exposure of the conductive strand is avoided and a secure, reliable electrical connection to the electrode at the distal end of the medical lead is provided. However, this conductor type and method of manufacturing electrodes is challenging for leads with thick walls of electrically insulating material.

Against the background described above, multi-filament leads are often specifically designed to match the combination of the electrical and mechanical requirements. But this means that any change in the requirements necessitates a new multi-filament lead design which is expensive, in particular if narrow tolerances are to be met as it is in a medical environment.

Document US 6,501,991 B1 discloses a multi-conductor lead body having improved mechanical characteristics and improved manufacturability, wherein the lead body includes multiple conductors wound around a generally cylindrical or tubular, insulating core member. In this configuration, the ability to adjust for varying flexibility and deformation characteristics of the implantable medical leads is poor.

Accordingly, it is an object of the invention to provide a cost-efficient implantable medical lead for different use cases which accommodates multiple electrically conducting filaments and has improved mechanical properties and excellent electrical insulation.

The above-mentioned object is solved by an implantable medical lead having the features of claim 1.

The object is solved by a an implantable medical lead comprising an electrically insulating tubular lead body having an internal lumen accommodating at least two conductors, wherein each conductor is configured to electrically connect one electrode located at the distal end of the lead and an electrical connector assembly at the proximal end of the lead, wherein each conductor comprises an electrically conductive core and an electrically insulating sleeve, wherein a cross section of the electrically insulating sleeve of at least one of the at least two conductors forms an inner circumference and an outer circumference, wherein the inner circumference and the outer circumference have a substantially elliptical shape exhibiting an eccentricity $\varepsilon$ in the range $0.4 \leq \varepsilon1 \leq 0.85$.

The electrically insulating tubular lead body of the inventive lead extends from the proximal end of the implantable medical lead (in the following: lead) to the distal end of the lead. At the distal end, at least two electrodes for sensing and/or therapy are provided, wherein each electrode is electrically connected to one specific terminal accommodated at the electrical connector assembly. In one embodiment, the medical lead may comprise a fixation assembly at its distal end to fix the distal tip of the lead at a pre-defined target location in the patient's body. The lead may be used in connection with electrical therapy like defibrillation, pacing, SCS, DBS, neurostimulation and/or in connection with electrical sensing of bodily parameters. For that, the electrical connector assembly may be electrically and mechanically detachably connected with a pulse generator and/or a sensing and/or data processing device. Electrical signals from the pulse generator and/or the sensing and/or data processing device may be transmitted via the lead to the at least two electrodes and/or sensed electrical signals from the electrodes may be transmitted via the lead to the pulse generator and/or the sensing and/or data processing device. Accordingly, in one embodiment, the electrical connector assembly is configured to be connected to a connector of a signal generator and/or a sensing and/or data processing device.

The electrically insulating tubular lead body is a tubular structure consisting of an electrically insulating material and mechanically protecting the at least two conductors accommodated within the internal lumen of the tubular lead body. The internal lumen of the tubular lead body accommodates at least two conductors, wherein each electrode is electrically connected to one terminal of the electrical connector assembly by a conductor. Each conductor comprises an electrically conductive core and an electrically insulating sleeve. The connector core may be formed, for example, by one electrically conducting rope-shaped strand, by at least two braided or wound strands, by a helical strand or by a rope-shaped central strand and a coil member wound around the central strand. Each conductor of the at least two conductors may be designed differently from the other one(s) of the at least two conductors. The design of each of the at least two conductors depends on the purpose of the implantable medical lead. For example, a conductor having a core formed by a coil member wound around a central strand is usually used for applications using high voltages (e.g. defibrillation signals). The electrically insulating sleeve of each conductor fully surrounds the electrically conducting core to provide electrical isolation with regard to the electrically conducting core of each other conductor. Each one of the at least two conductors extends from the proximal end of the tubular lead body to its distal end. The at least two conductors are disposed adjacently in radial direction of the tubular lead body. The tubular lead body fully encompasses all conductors with the exception of the connection area of the electrical connector assembly at the proximal end of the tubular lead body and the area where the electrodes are located at the distal end of the tubular lead body.

The internal lumen of the electrically insulating tubular lead body accommodates at least two conductors, wherein a cross section of the electrically insulating sleeve of at least one of the at least two conductors forms an inner circumference and an outer circumference, wherein the inner circumference and the outer circumference have an elliptical shape. In contrast to circular shaped inner and out circumferences of the cross section of the electrically insulating sleeve, an elliptical shape allows a greater variation of the mechanical properties caused by the specific ratio of cavity and wall thickness based on the chosen eccentricity. For example, a greater eccentricity provides a considerable cavity around a predefined, in its cross section usually circularly formed electrically conductive rope-shaped core adding compression volume and flexibility due to the shape difference between the conductive core having a circular profile and the electrically insulating sleeve having an elliptical profile. Accordingly, an additional electrically insulating (empty) tube may be omitted. In addition, elliptical shapes have unequal geometric moments of inertia with respect to the principal axis. Thereby, the mechanical properties may be improved, particularly with regard to a preferred direction of deformation under a specific mechanical load.

When viewing the cross section of the electrically insulating sleeve of the at least one conductor of the at least two conductors, both, the inner circumference and the outer circumference form a substantially elliptical curve. An ellipse is a plane curve surrounding two focal points such that for all points on the curve, the sum of the two distances to the focal points is constant. With regard to the center point of the ellipse, the ellipse comprises a major axis *a* and a minor axis *b*. The eccentricity $\varepsilon$ is defined as follows, wherein *a* > *b*:

$$\varepsilon = \sqrt{1 - \left(\frac{b}{a}\right)^2}$$

The eccentricity of the electrically insulating sleeve of at least one of the at least two conductors $\varepsilon$ is for example in the range $0.4 \leq \varepsilon 1 \leq 0.85$, in particular in the range $0.5 \leq \varepsilon 1 \leq 0.6$. The eccentricity of the inner circumference and the outer circumference may be identical or different, wherein, for example, the eccentricity of the outer circumference is smaller than the eccentricity of the inner circumference of the cross section of the electrically insulating sleeve.

For the implantable medical lead according to the invention, a separate electrically insulating sleeve is used for each one of the used electrically conducting supply lines which accordingly forms the core of the respective conductor. The resulting bundle of conductors is held together by the larger outer electrically insulating tubular lead body. For enhanced mechanical properties, at least one of the at least two conductors exhibits the above-explained eccentricity. The insulating sleeves of the conductors increase the electrical insulation. The eccentric at least one insulating sleeve adds compression volume for enhanced flexibility of the lead. The modular concept based on simple conductors ensures a flexible provision of lumens for signal lines that can be arranged as needed and quickly adapted to changes in the diameter or design/construction of each one of the signal lines. In addition, the design offers a simple way to arrange the conductors in a spiral. As indicated above, the elliptical shape of the at least one of the at least two conductors offers improved mechanical properties and reduced time and cost of materials. The eccentricity of the elliptical shape of the electrically insulating sleeve may be adapted based on the needed compression volume and the cross-sectional shape of the electrically conducting core of the respective conductor.

The wall thickness of the electrically insulating sleeve of one conductor is, for example, in the range between 0.025 mm and 0.3 mm. The diameter of the electrically conductive core of one conductor is, for example, in the range between 0.1 mm and 0.6 mm. The major axis of the outer circumference of the electrically insulating sleeve of one conductor is, for example, at least 0.2 mm and may vary between 0.5 mm and 1 mm. The minor axis of the outer circumference of the electrically insulating sleeve of one conductor is, for example, 1 mm at maximum and may vary between 0.15 mm and 0.5 mm. The major axis of the inner circumference of the electrically insulating sleeve of one conductor is, for example, at least 0.15 mm and may vary between 0.6 mm and 0.8 mm. The minor axis of the inner circumference of the electrically insulating sleeve of one conductor is, for example, 0.6 mm at maximum and may vary between 0.1 mm and 0.5 mm.

In one embodiment, the lumen of the electrically insulating tubular lead body accommodates at least two conductors as described above and additionally at least one electrically insulating tube, wherein the electrically insulating tube has an empty inner lumen. The at least two conductors and the at least one electrically insulating tube are accommodated adjacently in radial direction and the whole bundle is covered by the electrically insulating tubular lead body. The cross section of at least one electrically insulating tube forms an inner circumference and an outer circumference, wherein the inner circumference and the outer circumference have a substantially elliptical shape. The at least one electrically insulating tube separates adjacent conductors. The electrically insulating tube increases the electrical insulation between the at least two conductors and increases the mechanical resistance. The at least one electrically insulating tube is used to fill the spaces between the conductors. At the same time, this increases the radial elasticity of the whole bundle and allows the conductors to give way radially to external loads, e.g. pressure and bending. As before, the preferred dislocation under mechanical loads and the extent of the compression volume can be adjusted by the eccentricity of the electrically insulating tube. The eccentricity $\varepsilon 2$ of the at least one electrically insulating tube is, for example, in the range $0.4 \leq \varepsilon 2 \leq 0.95$ in particular in the range $0.5 \leq \varepsilon 2 \leq 0.6$. The wall thickness of the electrically insulating tube is, for example, in the range between 0.025 mm and 0.3 mm. The major axis of the outer circumference of the electrically insulating tube is, for example, at least 0.1 mm and may vary between 0.2 mm and 0.5 mm. The minor axis of the outer circumference of the electrically insulating tube is, for example, 1 mm at maximum and may vary between 0.05 mm and 0.5 mm. The major axis of the inner circumference of the electrically insulating tube is, for example, at least 0.1 mm and may vary between 0.2 mm and 0.8 mm. The minor axis of the inner circumference of the electrically insulating tube is, for example, 0.6 mm at maximum and may vary between 0.025 mm and 0.2 mm.

The term "substantially" with regard to the eccentricity of the inner or outer circumference of the electrically insulating sleeve or the electrically insulating tube means that the curve at the inner circumference and the curve at the outer circumference of the electrically insulating sleeve or the electrically insulating tube may comprise minor deviations from the ellipse or its defined eccentricity due to the fact that it is not a mathematical but a real object. For example, more than 80%, particularly more than 90% of the respective curve at the inner circumference and/or of the respective curve at the outer circumference may have the respective, above-defined elliptical shape.

In one embodiment, the at least two conductors may comprise at least one conductor wherein its electrically insulating sleeve has an annular cross section. Accordingly, at least one conductor having an electrically insulating sleeve of elliptical cross section (as described above) and at least one conductor having an electrically insulating sleeve of annular cross section are combined in one bundle. Accordingly, in one embodiment, the at least one electrically insulating tube may have an annular cross section. Alternatively, a plurality of electrically insulating tubes may comprise tubes having an annular cross section and tubes having an elliptical cross section (as described above).

In one embodiment, the at least two conductors and, if applicable, the at least one electrically insulating tube accommodated within the lumen of the electrically insulating tubular lead body are wound around each other. The at least two conductors and, if applicable, the at least one electrically insulating tube are wound clock-wise or counter clock-wise. Such wound bundle has a better flexibility when bent than a bundle of conductors and, if applicable, of at least one electrically insulating tube running in parallel.

During manufacturing of the implantable medical lead, the wound or parallel running bundle of the at least two conductors and, if applicable, of the at least one electrically insulating tube is introduced into the electrically insulating tubular lead body and advanced along the longitudinal axis of and within the tubular lead body until the tubular lead body covers the bundle. Prior introducing the wound bundle into the tubular lead body, the bundle may additionally be fixed by laminating or overmolding, using, e.g. at least one material of the group comprising silicone, polyurethane, polyethylene, polyimide, parylene. For assembling the bundle of tubular elements and the lumen of the electrically insulating tubular lead body, the lead body may be heated to increase its inner diameter. Afterwards the bundle is inserted, and the lead body is shrunk onto it.

In one embodiment, the bundle may comprise at least four, five, six or more than six conductors and at least two, three, four or more than four electrically insulating tubes. In one embodiment, the number of (empty) electrically insulating tubes providing additional compression volume may be smaller than the number of conductors of one bundle.

In one embodiment, the implantable medical lead further comprises an electrically insulating core member, wherein the at least two conductors and, if applicable, the at least one electrically insulating tube are wound around the core member. Accordingly, the core member forms one element of the bundle comprising the conductors and, if applicable, the at least one electrically insulating tube. For example, the central core member may be solid (i.e. filled) or reinforced using an additional central strand. Alternatively, the core member has a tubular form having an inner lumen. The additional central strand may comprise at least one material of the group comprising silicone, polyurethane, polyethylene, polyimide, polyamide, polytetrafluoroethylene. Such configuration increases the mechanical resistance of the implantable medical lead even further. In one embodiment, the outer shape of the cross section of the core member is circular. The outer diameter of the core member is, for example, at least 0.5 mm and/or may be in the range, for example, between 0.5 mm and 0.8 mm. If the core member has a tubular shape, the wall thickness of the core member may have, for example, a maximum value of 0.3 and/or may vary, for example, between 0.025 mm and 0.1 mm.

In one embodiment, a distal end of the electrically conductive core of the at least one of the at least two conductors is electrically connected to a crimping sleeve. This is possible, since the wall thickness of the electrically insulating material is reduced compared to the state of the art design. Hence, the manufacturing process for an electrode portion can be provided in less time and with reduced costs.

In one embodiment, the electrically conductive core of at least one of the at least two conductors is formed by at least two strands. These strands may be, for example, braided to reduce mechanical stress and increase the resistance against damage. It is advantageous that any type of core can be used within one conductor of the implantable medical lead. In one embodiment the electrically conductive core of at least one of the at least two conductors is formed by a central strand and a coil member wound around the central strand. The central strand may comprise at least one material of the group comprising MP35N, platinum, silver, iridium, gold, copper, and/or an alloy or combination of the aforementioned. The coil member may comprise at least one material of the group comprising MP35N, platinum, silver, iridium, gold, copper, and/or an alloy or combination of the aforementioned. The last mentioned configuration is especially beneficial if a low electrical resistance core is required, for, e.g., transmission of high voltage defibrillator signals.

In one embodiment, a clearance within the internal lumen of the electrically insulating tubular lead body is filled with electrically insulating material (e.g., silicone, polyurethan, butyl) for further electrical insulation.

In one embodiment, the electrically insulating tubular lead body, the at least one electrically insulating tube and/or the electrically insulating sleeve comprises thermoplastic or thermosetting material, e.g. at least one material of the group comprising silicone, polyurethane. In one embodiment, the material of the electrically insulating tubular lead body, of the at least one electrically insulating tube and/or of the electrically insulating sleeve may be a flexible material. In one embodiment, the electrically conducting core of one conductor of the at least two conductors comprises electrically conducting material, e.g. at least one material of the group comprising MP35N, platinum and/or silver.

The present invention will now be described in further detail with reference to the accompanying schematic drawings, wherein

Fig. 1     shows a first embodiment of an implantable medical lead in a side view,

Fig. 2     depicts a cross section along the line A-A of the embodiment of Fig. 1,

Fig. 3     illustrates a longitudinal section of a second embodiment of the implantable medical lead,

Fig. 4     shows a cross section of a third embodiment of the implantable medical lead,

Fig. 5     depicts a cross section of a fourth embodiment of the implantable medical lead, and

Fig. 6     illustrates a cross section of a fifth embodiment of the implantable medical lead.

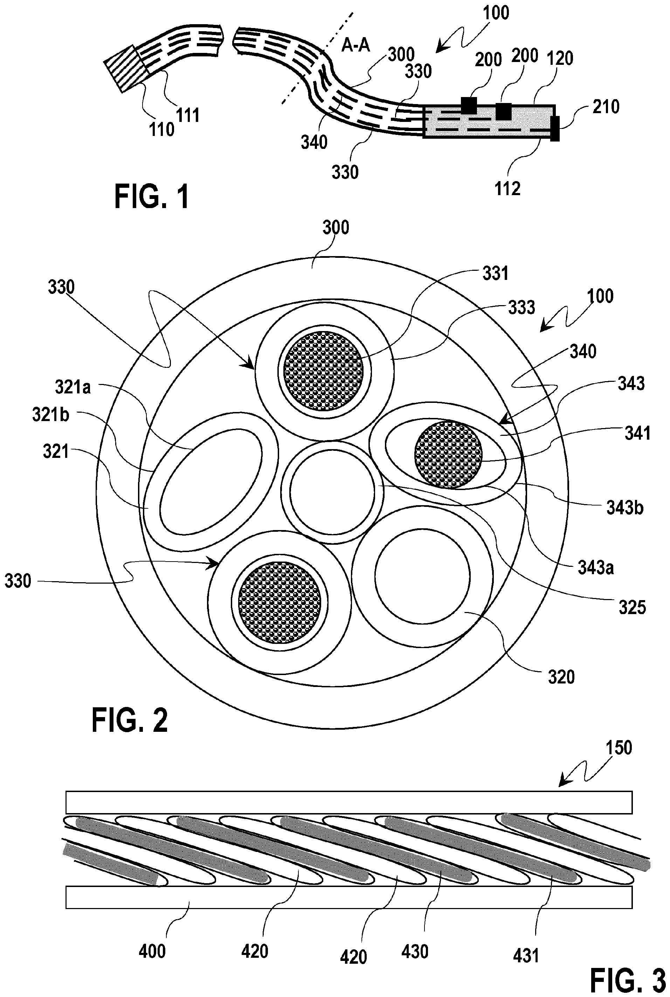

Fig. 1 shows a first embodiment of an implantable medical lead 100 having a proximal end 111 and a distal end 112. The implantable medical lead 100 electrically and mechanically connects an electrical connector assembly 110 located at the proximal end 111 to electrodes 200, 210 located at the distal end 112. Each electrode 200, 210 is electrically coupled via a conductor 330, 340 to a respective terminal at the electrical connector assembly 110 that is configured to couple the implantable medical lead 100 to a signal generator and/or sensing and/or data processing device to provide and/or receive electrical signals to/from the respective electrode 200, 210. The electrodes 200 are accommodated, for example at a shell surface of a fixation assembly 120 located at the distal end 112 of the implantable medical lead 100. At the distal tip of the fixation assembly 120, an electrode 210 formed as a fixation member (e.g. a helical member) may be provided for fixing the distal tip of the implantable medical lead 100 at a pre-defined target location within the patient's body, for example, a heart's tissue.

Fig. 2 shows the first embodiment of the implantable medical lead 100 in a cross section. The implantable medical lead 100 comprises two first conductors 330 having a circular cross section with a single-filament electrically conducting core 331 with a circular cross section covered by an electrically insulating sleeve 333. Additionally, the implantable medical lead 100 comprises a second conductor 340 having an electrically conducting core 341 with a circular cross section and an electrically insulating sleeve 343 exhibiting an elliptical shape in its cross section. The material of the electrically conducting cores 331, 341 is, for example, MP35N, platinum, silver, etc.

As one can derive from Fig. 2 the electrically insulating sleeve 343 forms an inner circumference 343a and an outer circumference 343b, wherein both, the inner circumference 343a and the outer circumference 343b have a substantially elliptical shape with an eccentricity $\varepsilon1$ of, for example 0.5.

The implantable medical lead 100 further comprises two electrically insulating tubes 320, 321 with empty lumen for compression. The first electrically insulating tube 320 has an annual cross section and the second electrically insulating tube 321 forms an inner circumference 321a and an outer circumference 321b, wherein both, the inner circumference 321a and the outer circumference 321b have a substantially elliptical shape with an eccentricity $\varepsilon2$ of, for example, 0.7.

The electrically insulating material of the electrically insulating sleeves 333, 343 of conductors 330, 340 and of the electrically insulating tubes 320, 321 may consist of, for example, silicone (SI) or polyurethane (PU). The electrically insulating sleeve 333 of the first conductor 330 may have an inner diameter of 0.5 mm and an outer diameter of 0.55 mm. The major axis (with regard to the center point of the ellipse) of the ellipse formed by the inner circumference 343a of the electrically insulating sleeve 343 of the second conductor 340 is, for example, 0.3 mm and the minor axis of the ellipse formed by the inner circumference 343a of the electrically insulating sleeve 343 of the second conductor 340 is, for example, 0.2 mm. The wall thickness of the electrically insulating sleeve 343 is, e.g., 0.05 mm. The major axis (with regard to the center point of the ellipse) of the ellipse formed by the inner circumference 321a of the electrically insulating tube 321 is, for example, 0.35 mm and the minor axis of the ellipse formed by the inner circumference 321b of the electrically insulating tube 321 is, for example, 0.25 mm. The wall thickness of the electrically insulating tube 321 is, e.g., 0.05 mm. Within each of the internal lumens of the sleeves 333, 343 of conductors 330, 340 a rope-shaped electrically conducting core 331, 341 with a diameter of 0.4 mm having a circular cross section is located.

The conductors 330, 340 and the tubes 320, 321 of the implantable medical lead 100 are grouped in the form of a tight packing around an electrically insulating and tubular core member 325 with an inner diameter of 0.43 mm and an outer diameter of 0.48 mm. The conductors 330, 340, the tubes 320, 321 and the electrically insulating core member 325 form a bundle. The bundle may be twisted by rotating the conductors 330, 340 and the electrically insulating sleeves 320, 321 around the central axis (e.g. the axis of the core member 325), thus reducing the outer diameter of the tube bundle (see Fig. 2). In case the sleeves 333, 343 and tubes 320, 321 consist of plastic material such as SI or PU, the adhesive forces between the sleeves 333, 343 and tubes 320, 321 are sufficient to maintain the twisted state for a short time. The (twisted) bundle is covered with an outer electrically insulating tubular lead body 300 having an annular cross section with, e.g., an inner diameter of 2 mm and an outer diameter of 2.3 mm.

Fig. 3 depicts another embodiment of an implantable medical lead 150 having conductors 430, 431 and electrically insulating tubes 420 that are wound around an inner electrically insulating core (not shown). As one can derive from the longitudinal section of Fig. 3 one conductor 430, 431 alternates with one electrically insulating tube 420 within the wound bundle. Regarding the further properties of this embodiment it is referred to the first embodiment of an implantable medical lead 100. The conductors 430, 431 correspond to the conductors 330, 340 regarding their dimensions and specific structure, wherein the specific structure generally includes the specific shape. The electrically insulating tube 420 corresponds to the electrically insulating tube 320 regarding its dimensions and specific structure. The same applies to the not-shown electrically insulating core - it corresponds to the electrically insulating core 325 of the first embodiment. Accordingly, the electrically insulating tubular lead body 400 of the second embodiment basically corresponds to the electrically insulating tubular lead body 300 but having a smaller inner and outer diameter.

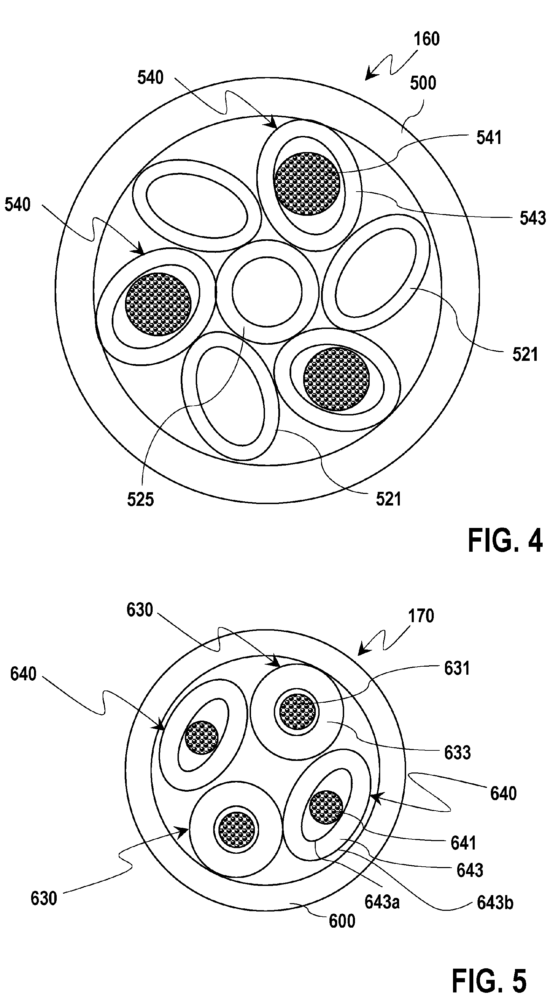

Fig. 4 shows a third embodiment of an implantable medical lead 160 having three conductors 540, each consisting of an electrically conducting core 541 and an electrically insulating sleeve 543. Each conductor 540 has dimensions and specific structure comparable to the second conductor 340 of the first embodiment. Further, the implantable medical lead 160 comprises three electrically insulating tubes 521 having dimensions and specific structure comparable to the tubes 321 of the first embodiment. The conductors 540 and the electrically insulating tubes 521 are wound around an electrically insulating core member 525 having a annular cross-sectional shape comparable to the core member 325 of the first embodiment but a greater wall thickness. The elliptical deformation of the electrically insulating sleeves 543 and the electrically insulating tubes 521 when the bundle is twisted (see Fig. 4) results in a maximum outer diameter that allows the bundle to be over-fitted with an electrically insulating tubular lead body 500 having an inner diameter of 2 mm.

Fig. 5 depicts an embodiment of an implantable medical lead 170 omitting an electrically insulating core member and an electrically insulating tube (with empty inner lumen providing compression volume). This embodiment comprises two first conductors 630, each with an electrically conducting core 631 and an electrically insulating sleeve 633, wherein the electrically insulating sleeve 633 has an annular cross section. Alternating, along an inner circular line along its cross section the implantable medical lead 170 comprises two second conductors 640, each with an electrically conducting core 641 and an electrically insulating sleeve 643, wherein the electrically insulating sleeve 643 has an elliptical cross section. The electrically insulating sleeve 643 of each second conductor 640 forms an inner circumference 643a and an outer circumference 643b, wherein both, the inner circumference 643a and the outer circumference 643b have a substantially elliptical shape with an eccentricity $\varepsilon 1$ of, for example, 0.7. The bundle of wound first conductors 630 and second conductors 640 is covered by an electrically insulating tubular lead body 600. As one can derive from Fig. 5, the internal lumen of the electrically insulating sleeves 643 having an elliptical cross section provides clearances forming considerable compression volume that is located on two sides in radial direction of the respective electrically conducting core 641 so that an additional electrically insulating tube with empty lumen is not necessary.

One embodiment for assembly of such an implantable medical lead 170 is to connect the electrically conducting cores 631, 641 to respective terminals of an electrode connector assembly and to guide the individual electrically insulating sleeves 633, 643 over the distal ends of one of the cores 631, 641 and twist them. The twisted conductors 630, 640 with electrode connector assembly may then be pulled through the outer electrically insulating tubular lead body 600 by means of the electrode connector assembly.

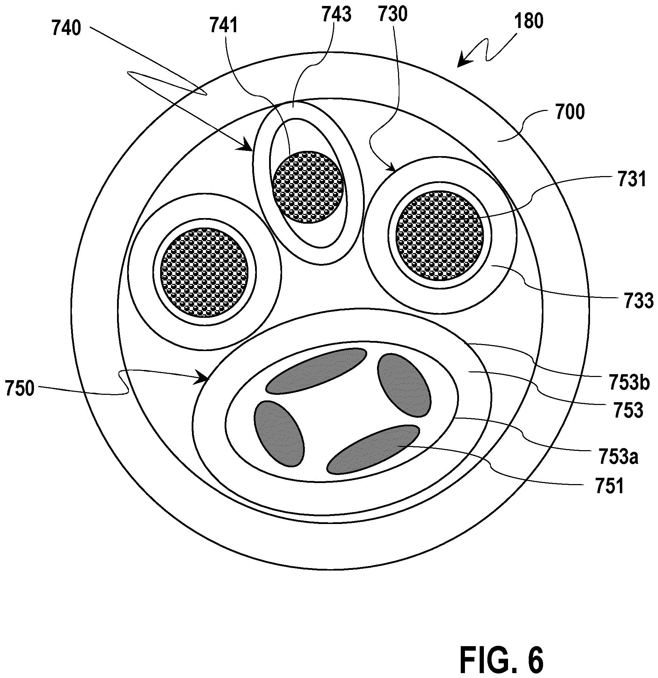

The fifth embodiment of an implantable medical lead depicted in Fig. 6 comprises two first conductors 730, one second conductor 740 and one third conductor 750. Each one of the first conductors having a rope-shaped electrically conducting core 731 and an electrically insulating sleeve 733 is similar to the first conductor 330 of the first embodiment regarding its specific structure and dimensions. Similarly, the second conductor 740 corresponds to the second conductor 340 of the first embodiment with respect to its specific structure and dimensions, wherein the second conductor 740 comprises a rope-shaped electrically conducting core 741 and an electrically insulating sleeve 743. The third conductor 750 comprises a helical core 751 with an outer diameter of 0.7 mm accommodated within an electrically insulating sleeve 753. The electrically insulating sleeve 753 forms an inner circumference 753a and an outer circumference 753b, wherein both, the inner circumference 753a and the outer circumference 753b have a substantially elliptical shape with an eccentricity $\varepsilon 1$ of, for example, 0.47. Further, the major axis (with regard to the center point of the ellipse) of the ellipse formed by the inner circumference 753a of the electrically insulating sleeve 753 of the third conductor 750 is, for example, 0.4 mm and the minor axis of the ellipse formed by the inner circumference 753a of the electrically insulating sleeve 753 of the third conductor 750 is, for example, 0.3 mm. The wall thickness of the electrically insulating sleeve 753 is, e.g., 0.15 mm. The four individual conductors 730, 740, 750 are inserted into an electrically insulating tubular lead 700 body with an inner diameter of 1.6 mm and an outer diameter of 1.9 mm.

In one embodiment, the bundle of individual conductors and electrically insulating tubes (having an empty lumen) is inserted into an electrically insulating tubular lead body and the lead body is shrunk onto the conductors and tubes like a heat-shrinkable sleeve.

The above implantable medical leads 100, 150, 160, 170, 180 may be easily and cost-effective designed according to the specific electrical and mechanical requirements. Further, these leads exhibit excellent mechanical properties such as enhanced radial elasticity.

## Claims

1. An implantable medical lead (100, 150, 160, 170, 180) comprising an electrically insulating tubular lead body (300, 400, 500, 600, 700) having an internal lumen accommodating at least two conductors (330, 340, 430, 431, 540, 630, 640, 730, 740, 750), wherein each conductor (330, 340, 430, 431, 540, 630, 640, 730, 740, 750) is configured to electrically connect one electrode (200, 210) located at the distal end (112) of the lead and an electrical connector assembly (110) at the proximal end (111) of the lead, wherein each conductor (330, 340, 430, 431, 540, 630, 640, 730, 740, 750) comprises an electrically conductive core (331, 341, 541, 631, 641, 731, 741) and an electrically insulating sleeve (333, 343, 543, 633, 643, 733, 743, 753), wherein a cross section of the electrically insulating sleeve (343, 543, 643, 743, 753) of at least one conductor (340, 540, 640, 740, 750) of the at least two conductors (330, 340, 430, 431, 540, 630, 640, 730, 740, 750) forms an inner circumference (343a, 643a, 753a) and an outer circumference (343b, 643b, 753b), wherein the inner circumference (343a, 643a, 753a) and the outer circumference (343b, 643b, 753b) have a substantially elliptical shape exhibiting an eccentricity $\varepsilon$ in the range $0.4 \leq \varepsilon 1 \leq 0.85$.

2. The implantable medical lead according to claim 1 comprising additionally at least one electrically insulating tube (320, 321, 521), wherein a cross section of the electrically insulating tube (320, 321, 521) forms an inner circumference (321a) and an outer circumference (321b), wherein the inner circumference (321a) and the outer circumference (321b) have a substantially elliptical shape exhibiting an eccentricity ε in the range $0.4 \leq \varepsilon 2 \leq 0.95$.

3. The implantable medical lead according to any one of the aforementioned claims, wherein the at least two conductors (330, 340, 430, 431, 540, 630, 640, 730, 740, 750) and, if applicable, the at least one electrically insulating tube (320, 321, 521) are wound around each other.

4. The implantable medical lead according to any of the claims 1 to 2, further comprising an electrically insulating core member (325, 525), wherein the at least two conductors (330, 340, 430, 431, 540) and, if applicable, the at least one electrically insulating tube (320, 321, 521) are wound around the core member (325, 525).

5. The implantable medical lead according to any one of the aforementioned claims, wherein the core member (325, 525) is a tubular member.

6. The implantable medical lead according to any one of the aforementioned claims, wherein a distal end of the electrically conductive core (331, 341, 541, 631, 641, 731, 741) of the at least one of the at least two conductors (330, 340, 430, 431, 540, 630, 640, 730, 740, 750) is electrically connected to a crimping sleeve.

7. The implantable medical lead according to any one of the aforementioned claims, wherein the electrically conductive core of at least one of the at least two conductors (330, 340, 430, 431, 540, 630, 640, 730, 740, 750) is formed by at least two braided strands.

8. The implantable medical lead according to any one of the aforementioned claims, wherein the electrically conductive core of at least one of the at least two conductors (330, 340, 430, 431, 540, 630, 640, 730, 740, 750) is formed by a central strand and a coil member wound around the central strand.

9. The implantable medical lead according to any one of the aforementioned claims, wherein the electrical connector assembly (110) is configured to be connected to a connector of a signal generator and/or a data processing device.

10. The implantable medical lead according to any one of the aforementioned claims, wherein a clearance within the internal lumen of the electrically insulating tubular lead body (300, 400, 500, 600, 700) is filled with electrically insulating material, in particular silicone, polyurethane and/or butyl.

11. The implantable medical lead according to any one of the aforementioned claims, wherein the electrically conductive core (331, 341, 541, 631, 641, 731, 741) of at least one of the at least two conductors (330, 340, 430, 431, 540, 630, 640, 730, 740, 750) comprises at least one material of the group comprising MP35N, platinum and/or silver.

12. The implantable medical lead according to any one of the aforementioned claims, wherein the electrically insulating tubular lead body (300, 400, 500, 600, 700), the at least one electrically insulating tube (320, 321, 521) and/or the electrically insulating sleeve (333, 343, 543, 633, 643, 733, 743, 753) comprises at least one material of the group comprising silicone, polyurethane and/or butyl.

FIG. 1

FIG. 2

FIG. 3

540

160

500

541

543

521

540

521

525

**FIG. 4**

630

170

640

631

633

640

641

643

630

643a

643b

600

**FIG. 5**

FIG. 6

## EP 4 717 302 A1

Europäisches
Patentamt

European
Patent Office

Office européen
des brevets

# EUROPEAN SEARCH REPORT

**Application Number**

EP 24 20 2738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/204767 A1 (ZHAO YONG D [US]) 12 August 2010 (2010-08-12) | 1-9,11, 12 | INV. A61N1/375 H01B7/04 |
| Y | * paragraphs [0001], [0064], [0132], [0199], [0215] - [0221]; figures 1,6E,7A * | 10 | |
| Y | ----- US 2015/025611 A1 (LIM LILY [US] ET AL) 22 January 2015 (2015-01-22) * paragraph [0052]; figure 11 * ----- | 10 | ADD. A61N1/36 A61N1/05 H01B7/18 |
| A | US 2006/200216 A1 (CALZADA JAVIER E [US] ET AL) 7 September 2006 (2006-09-07) * paragraph [0045] * ----- | 1-12 | |

|  |
|---|
| **TECHNICAL FIELDS SEARCHED (IPC)** |
| A61N H01B |

The present search report has been drawn up for all claims

| Place of search | Date of completion of the search | Examiner |
|---|---|---|
| Munich | 24 January 2025 | Rogala, Tomasz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 2738

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2025

| Patent document cited in search report | Publication date | Patent family member(s) | | Publication date |
|---|---|---|---|---|
| US 2010204767 A1 | 12-08-2010 | NONE | | |
| US 2015025611 A1 | 22-01-2015 | US | 2013138188 A1 | 30-05-2013 |
| | | US | 2015025611 A1 | 22-01-2015 |
| | | US | 2016067475 A1 | 10-03-2016 |
| US 2006200216 A1 | 07-09-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6501991 B1